Europäisches Patentamt

European Patent Office

Office européen des brevets

(11) Publication number: **0 636 740 A1**

## EUROPEAN PATENT APPLICATION
published in accordance with Art. 158(3) EPC

(21) Application number: 94906367.1

(22) Date of filing: **10.02.94**

(86) International application number:
**PCT/JP94/00199**

(87) International publication number:
**WO 94/19527 (01.09.94 94/20)**

(51) Int. Cl.6: **D06M 16/00**, C12N 9/42

(30) Priority: **18.02.93 JP 51306/93**

(43) Date of publication of application:
**01.02.95 Bulletin 95/05**

(84) Designated Contracting States:
**DE DK ES FR IT**

(71) Applicant: **MEIJI SEIKA KABUSHIKI KAISHA**
**4-16 Kyobashi 2-chome,**
**Chuo-ku**
**Tokyo 104 (JP)**
Applicant: **Rakuto Kasei Industrial Co., Ltd.**
**5-1, Sekinotsu 4-chome, Otsu-shi**
**Shiga 520 (JP)**

(72) Inventor: **KAWAHATA, Keiko Seibutsu Kagaku**
**Kenkyusho**
**Meiji Seika Kabushiki Kaisha**
**3-1, Chiyoda 5-chome**
**Sakado-shi**
**Saitama 350-02 (JP)**
Inventor: **HAMAYA, Toru Seibutsu Kagaku**
**Kenkyusho**
**Meiji Seika Kabushiki Kaisha**
**3-1, Chiyoda 5-chome**

**Sakado-shi**
**Saitama 350-02 (JP)**
Inventor: **HIRAYAMA, Masao Seibutsu Kagaku**
**Kenkyusho**
**Meiji Seika Kabushiki Kaisha**
**3-1, Chiyoda 5-chome**
**Sakado-shi**
**Saitama 350-02 (JP)**
Inventor: **KONO, Toshiaki Seibutsu Kagaku**
**Kenkyusho**
**Meiji Seika Kabushiki Kaisha**
**3-1, Chiyoda 5-chome**
**Sakado-shi**
**Saitama 350-02 (JP)**
Inventor: **OTSUKA, Masaaki Rakuto Kasei**
**Industrial Co., Ltd.**
**5-1 Sekinotsu 4-chome**
**Otsu-shi**
**Shiga 520 (JP)**

(74) Representative: **Kyle, Diana**
**Elkington and Fife**
**Prospect House**
**8 Pembroke Road**
**Sevenoaks, Kent TN13 1XR (GB)**

(54) **CELLULASE PREPARATION AND METHOD OF TREATING CELLULOSIC FIBER THEREWITH.**

(57) A cellulose preparation which can improve the color and hand of cellulosic fibers while suppressing a lowering in the strengths of the fibers. The cellulose of the preparation can adsorb crystalline cellulose and has an avicellase activity to CMCase activity ratio of 0.1 or above. The fibers treated with this preparation can retain a relative residual tensile strength of at least 75 % per count at a weight reduction rate of 5 %.

EP 0 636 740 A1

## BACKGROUND OF THE INVENTION

Technical Field

The present invention relates to enzyme preparations comprising cellulase, i.e., cellulase preparations, and a method for treating cellulosic fibers using the same. More particularly, the present invention is concerned with cellulase preparations which can improve color and feeling of cellulosic fibers while preventing a lowering in strength of the cellulosic fibers.

Background Art

It is widely known that treatment of cellulosic fibers with an aqueous solution containing cellulase can improve color, feeling and other various properties of the cellulosic fibers. This treatment is called "loss in weight treatment," or "stone-free stone-washed process". Moreover, the cellulosic fibers, which have been subjected to the above treatment, is called "stone-washed denim like fibers."

For example, preparations for the treatment are proposed in Japanese Patent Laid-Open Nos. 174298/1987, 8287/1992 and 500702/1992, and methods for the treatment are proposed in Japanese Patent Laid-Open No. 185775/1992, Japanese Patent Publication No. 19235/1990, Japanese Patent Laid-Open Nos. 80680/1990, 167378/1991 and 80673/1990, Japanese Patent Publication No. 13471/1992, Japanese Patent Laid-Open Nos. 167366/1991, 241076/1991 and 241077/1991 and Japanese Patent Publication Nos. 20751/1990 and 13470/1992.

In general, however, it is pointed out that the treatment of the cellulosic fibers with cellulase unfavorably lowers the strength of the cellulosic fibers.

In order to prevent the lowering in strength of the cellulosic fibers, a proposal has been made, for example, on a method for shortening the treatment time (Japanese Patent Publication No. 48236/1977). So far as the present inventors know, however, this method is unsatisfactory in the improvement in properties of the fiber although it can prevent the lowering in strength of the fiber.

Further, various methods have been proposed for preventing the lowering in fiber strength. Examples of such methods known in the art include a method for carrying out a chemical pretreatment (for example, Japanese Patent Publication Nos. 23591/1989 and 40787/1990, Japanese Patent Laid-Open Nos. 216282/1990, 51376/1991 and 99084/1985), a method in which a resin or a salt is allowed to exist together with cellulase (for example, Japanese Patent Laid-Open No. 216283/1990 and 50380/1992), a method in which a chemical post treatment is carried out (for example, Japanese Patent Laid-Open No. 50365/1992), a method in which a pretreatment is carried out using plasma (for example, Japanese Patent Laid-Open No. 260067/1989), a method in which the cellulosic fiber is used in combination with a non-cellulosic fiber (for example, Japanese Patent Laid-Open Nos. 97972/1991 and 234876/1991) and a method in which a raising treatment is also carried out (for example, Japanese Patent Laid-Open No. 18174/1992). So far as the present inventors know, however, these methods too have room for improvement, i.e., are unsatisfactory in the effect of the treatment and provide feeling different from that inherently provided by the treatment with cellulase. Further, the addition of various chemicals may unfavorably cause damage to equipment and machinery. It also involves risk associated with operation and a new problem causing difficulty for the treatment of the waste water.

On the other hand, the use of cellulase preparations as an additive to detergents for clothing has been developed. This is based on finding that in the cellulase a component not having an adsorptivity to crystalline cellulose, particularly an endoglucanase component not having an adsorptivity to crystalline cellulose, apparently exhibits substantially no action on a highly crystalline region but acts on the fiber only in its non-crystalline region where soils are confined, thereby enabling the soils to be removed (Agric. Biol. Chem., 53(5), 1275-1281, 1989). In this case, the cellulase aims to act only on the non-crystalline region of cellulose to remove soils and, hence, cannot, of course, be expected to have the effect of improving the feeling or color of the cellulosic fiber.

Japanese Patent Laid-Open No. 264699/1988 describes that the cellulase has been evaluated in terms of the degree of crystallinity about cellulose (i.e., highly crystalline, non-crystalline or low crystalline property) and decomposability. This publication merely proposes a detergent composition for clothing. In fact, the cellulase cannot improve the feeling and color of cellulosic fibers.

Furthermore, it is also reported that an endoglucanase component adsorptive to crystalline cellulose is important to the treatment of cellulosic fibers (for example, Japanese Patent Laid-Open Nos. 23699/1982 and 504080/1991). This proposal is also on additives to detergents for clothing but not on preparations for loss in treatment and prevention of a lowering in strength of the fiber. In fact, the above cellulase cannot sufficiently improve the feeling and color of the fiber. Moreover, the treatment of the fiber with the cellulase causes a lowering in strength of the fiber. It is certainly pointed out that the strength of a fiber having a high crystallinity, such as cotton, is related to the state of the crystalline region ("Sen-i Butsurigaku" pp. 70-71: edited by The society of Fiber Science and Technology, Japan; and "Sen-i Binran Kakohen" pp. 1042-1043: edited by The society of Fiber Science and Technology, Japan). These facts, however, suggest that mere consideration of the endoglucanase component having an adsorptivity to a crystalline cellulose cannot prevent a lowering in strength of the fiber.

As described above, it can be said that in the method for improving the feeling and color of a cellulosic fiber using a cellulase, the task to be attained is to prevent a lowering in strength of the fiber. So far as the present inventors know, when cellulase preparations currently in use are applied to the treatment of the cellulosic fiber, the fiber strength (the relative residual tenacity per yarn yarn count) is unfavorably lowered to about 65% of that before the treatment in a loss in weight of 5%.

## SUMMARY OF THE INVENTION

Accordingly, an object of the present invention is to provide a cellulase preparation which can improve the color and feeling of a cellulosic fiber while preventing a lowering in strength of the cellulosic fiber.

The present inventors have now found that a particular activity ratio of a cellulase component which has an adsorptivity to crystalline cellulose is important to the treatment of a cellulosic fiber. The present invention has been made based on such finding.

Specifically, according to an aspect of the present invention, there is provided a cellulase preparation characterized in that when a fiber is treated with said cellulase preparation, the treated fiber has a relative residual tenacity of not less than 75% per yarn count or more in a loss in weight of 5%.

According to a preferred embodiment, the cellulase preparation of the present invention comprises a cellulase which has an adsorptivity to crystalline cellulose and an avicellase activity to CMCase activity ratio (Avicelase activity/CMCase activity) of not less than 0.1.

## BRIEF DESCRIPTION OF THE DRAWINGS

Fig. 1 is a diagram showing an elution pattern of a commercially available cellulase eluted using an anion-exchange column with gradient of a NaCl solution; and

Fig. 2 is a graph showing the relationship between the Avicelase activity/CMCase activity ratio and the residual tenacity of a cellulosic fiber when the cellulosic fiber has been treated with the cellulase preparation according to the present invention.

## DETAILED DESCRIPTION OF THE INVENTION

While cellulase is a generic term for enzyme having an activity to hydrolyze cellulose, the cellulase preparation according to the present invention comprises a cellulase having an adsorptivity to crystalline cellulose, and the component having an adsorptivity to crystalline cellulose has the ratio of Avicelase activity to CMCase activity not less than 0.1.

In the present invention, the presence of the component having an adsorptivity to crystalline cellulose in the cellulase preparation can be determined by a method described in the "Evaluation of adsorptivity to crystalline cellulose" which will be described later. Specifically, the cellulase is brought into contact with crystalline cellulose and divided into a component which is adsorbed to crystalline cellulose and a component which is not adsorbed but left in the supernatant under conditions which will be described in the "Evaluation of adsorptivity to crystalline cellulose" described below. The presence of the cellulase having an adsorptivity to crystalline cellulose can be confirmed by subtracting the protein content of the cellulase left in the supernatant from the protein content of the original cellulase.

In the present invention, the component having an adsorptivity to crystalline cellulose has an Avicelase activity to CMCase activity ratio (i.e., a value obtained by dividing the Avicelase activity (U/g) by the CMCase activity (U/g): hereinafter often referred to as "Avicelase activity/CMCase activity value") of not less than 0.1, preferably not less than 0.25, most preferably not less than 0.4.

The Avicelase activity may be measured, for example, by a method described in "Biosci. Biotech. Biochem., 56(10), 1523-1528, 1992 or a method described in "Shiryo Tenkabutsu No Seibun Kikaku To Shusaisho (A Book Describing Specifications for Components of Feed Additives), 5th edition," pp. 55-56 (published by Japan Scientific Feeds Association), and the CMCase activity may be measured, for example, by a method described in Pure & Appl. Chem., Vol. 59, No. 2, 257-268, 1987.

However, the presence of the component not having an adsorptivity to crystalline cellulose is not excluded from the cellulase preparation of the present invention for the reason which will be described later. Therefore, the Avicelase activity/CMCase activity value of the component having an adsorptivity to crystalline cellulose for a certain cellulase may be measured as follows. Specifically, for a certain cellulase, the Avicelase activity and the CMCase activity of the whole cellulase are measured. Separately, a component having an adsorptivity to crystalline cellulose is precipitated together with the crystalline cellulose under conditions described in the "Evaluation of adsorptivity to crystalline cellulose" described below so that a component not having an adsorptivity to the crystalline cellulose is allowed to exit in the supernatant. Then, the Avicelase activity and the CMCase activity of the component present in the supernatant are measured by the above method. The Avicelase activity and CMCase activity values of the component having an adsorptivity to crystalline cellulose are obtained by subtracting the Avicelase activity and CMCase activity values of the component present in the supernatant respectively from the Avicelase activity and CMCase activity values of the whole cellulase. The Avicelase activity/CMCase activity values of the component having an adsorptivity to crystalline cellulose can be obtained from the above values. The activity measuring method will be described later in more detail.

The cellulase component contained in the cellulase preparation according to the present invention is preferably purified as much as possible so that it can exhibit an adsorptivity to crystalline cellulose and an Avicelase activity/CMCase activity ratio of not less than 0.1. On the other hand, the present inventors have confirmed that the component not having an adsorptivity to crystalline cellulose neither inhibits the action of the component having an adsorptivity to crystalline cellulose nor causes a lowering in strength of the fiber. Furthermore, it is considered that when turbulence occurs in the crystalline structure due to the action of the component having an adsorptivity to crystalline cellulose, the presence of the component not having an adsorptivity to crystalline cellulose advantageously has a favorable effect on the action of the component having an adsorptivity to crystalline cellulose. Therefore, the presence of the component not having an adsorptivity to crystalline cellulose in the cellulase preparation according to the present invention is not absolutely excluded.

The Avicelase activity/CMCase activity value for the component not having an adsorptivity to crystalline cellulose is not particularly limited for the same reason as described above.

In the present invention, that the Avicelase activity/CMCase activity value is not less than 0.1 is intended to mean that the activity ratio is not less than 0.1 under temperature, pH and other conditions under which the cellulase preparation is actually used. At the view of this point, a cellulase preparation of the present invention contains a cellulase component having an adsorptivity to crystalline cellulase and the Avicelase activity/CMCase activity value of not less than 0.1 under conditions such as temperature or pH for actual use of the preparation, even though the Avicelase activity/CMCase activity value is less than 0.1 under a certain another condition.

When a cellulosic fiber is treated with the cellulase preparation of the present invention, the treated cellulosic fiber advantageously has satisfactorily improved feeling, color and other properties without causing a significant lowering in fiber strength. Specifically, in a loss in weight of 5% of fiber, the cellulosic fiber has a relative residual tenacity of not less than about 75% per yarn count, preferably not less than 85% per yarn count.

The fiber to which the cellulase preparation of the present invention is applicable is the so-called "cellulosic fiber," such as denim, cotton and hemp. These cellulosic fibers, which have been treated with the cellulase preparation of the present invention, have improved feeling, color and other properties. In particular, in denim, the stone-washed like process can be applied while satisfactorily maintaining the fiber strength even after the treatment.

The loss in weight of the fiber is defined by the following equation:

$$\text{Loss in weight (\%)} = \left[ \frac{\text{weight after treatment with no enzyme added}}{\text{weight before treatment with no enzyme added}} - \frac{\text{weight after enzyme treament}}{\text{weight before enzyme treatment}} \right] \times 100$$

4

The tenacity per yarn count is intended to mean that described on page 8 of "Sangyo Yo Sen-i Shizai Handobukku (Handbook of fiber Materials for Industry)" (Textile Machinery Society of Japan/Industrial Textile Committee). Specifically, in general, the tenacity is a value obtained by dividing a load needed to break a sample by pulling by the initial cross-sectional area. In the present invention, the value obtained by using the weight per unit length instead of the cross-sectional area in the above definition is designated as "tenacity per yarn count." In the present invention, the change in tenacity per yarn count of the fiber upon the treatment is evaluated in terms of the relative residual tenacity defined by the following equation:

Relative residual tenacity (%) = (tenacity after enzyme treatment/tenacity after treatment with no enzyme added) x 100

The cellulase preparation according to the present invention can be prepared by concentrating the component having an adsorptivity to crystalline cellulose and an Avicelase activity from the conventional cellulase. Specifically, it can be provided by using column chromatography or other means to remove a component having an Avicelase activity/CMCase activity value of less than 0.1 so as to isolate a fraction having an Avicelase activity/CMCase activity value of not less than 0.1. Further examples of the preparation method include a method which comprises isolating a gene coding for a cellulase having the above properties and providing the recombinant cellulase by a gene manipulation technology.

The temperature and pH at which the cellulase preparation of the present invention is employed can be properly determined depending upon the cellulase component used and are not particularly limited. Preferably, when the contemplated cellulase is provided from the conventional cellulase, it preferably has an adsorptivity to crystalline cellulose in an acidic or neutral region. The temperature at which the cellulase exhibits the activity is preferably in the range of from 30 to 80°C, still preferably in the range of from 40 to 70°C, most preferably in the range of form 45 to 65° C. The pH at which the cellulase exhibits the activity is preferably in the range of from 3 to 8, still preferably in the range of from 4 to 7.

According to a preferred embodiment, the cellulase preparation of the present invention is used under conditions of a temperature of preferably in the range of from 30 to 80°C , still preferably in the range of from 40 to 70°C, most preferably in the range of from 45 to 65°C and a pH of preferably in the range of from 3 to 8, still preferably in the range of from 4 to 7.

According to a preferred embodiment, the cellulase is derived from Trichoderma, has an adsorptivity to crystalline cellulose and an Avicelase activity/CMCase activity value of not less than 0.1 and further has the following properties:
- average molecular weight: about 59 kd (by SDS-PAGE),
- isoelectric point: about 3.8 to 4.2,
- Avicelase activity: not less than 100 units/g of the total solid matter, and
. optimum pH: about 4.2 to 4.8.

As described above, in the present invention, the cellulase can be Provided from the conventional cellulase which may be derived from animals, plants and microorganisms.

Examples of eukaryotic microorganism capable of producing a cellulase include:

*Arthrobys superbaus, Aspergillus aureus, Aspergillus flavipes, Aspergillus fumigatus, Aspergillus fuchuenis, Aspergillus nidulans, Aspergillus niger, Aspergillus oryzae, Aspergillus rugulosus, Aspergillus sojae, Aspergillus sydwi, Aspergillus tamaril, Aspergillus terreus, Aspergillus unguis, Aspergillus ustus, Takamine−Cellulase, Aspergillus saitoi, Botrytis cinerea, Botryodipiodia theobromae, Cladosporium cucummerinum, Cladosporium herbarum, Coccospora agricola, Curvuiaria lunata, Chaetomium thermophile var. coprophile, Chaetomium thermophile var. dissitum, Sporotrichum thermophile, Taromyces amersonii, Thermoascus aurantiacus, Humicola grisea var. thermoidea, Humicola insolens, Malbranchea puichella var. sulfurea, Myriococcum albomyces, Stilbella thermophile, Torula thermophila, Chaetomium globosum, Dictyosteiium discoideum, Fusarium sp., Fusarium bulbigenum, Fusarium equiseti, Fusarium lateritium, Fusarium lini, Fusarium oxysporum, Fusarium vasinfectum, Fusarium dimerum, Fusarium japonicum, Fusarium scirpi, Fusarium solani, Fusarium moniliforme, Fusarium roseum, Helminthosporium sp., Memnoniella echinata, Humicola fucoatra, Humicola grisea, Monilia sitophila, Monotospora brevis, Mucor pusillus, Mycosphaerella citrulina, Myrothecium verrcaria, Papulaspore sp., Mycosphaerella citrinul, Myrothecium capsulatum, Penicillium chrysogenum, Penicillium freguentana, Penicillium funicilosum, Penicillium janthinellum, Penicillium luteum, Penicillium piscarium, Penicillium soppi, Penicillium spinulosum, Penicillium turbaturn, Penicillium digitatum, Penicillium expansum, Penicillium pusitlum, Penicillium rubrum, Penicillium wortmanii, Penicillium wariabile, Pestalotia palmarum, Pestalotiopsis westerdijkii, Phoma sp., Schizophyllum commune, Scopulariopsis brevicaulis, Rhizopus sp., Sporotricum carnis, Sporotricum pruinosum, Stachybotrys*

atra, Torula sp., Trichoderma viride (reesei), Trichurus cylindricus, Verticillium albo atrum, Aspergillus cellulosae, Penicillium glaucum, Cunninghamella sp., Mucor mucedo, Rhyzopus chinensis, Coremiella sp., Karlingia rosea, Phytophthora cactorum, Phytophthora citricola, Phytophthora parasitica, Pythium sp., Saprolegnia ceae, Seratocystis ulmi, Chaetomium globosum, Chaetomium indicum, Neurospora crassa, Sclerotium rolfsii, Aspergillus sp., Chrysosporium lignorum, Penicillium notatum, Pyricularia oryzae, Collybia veltipes, Coprinus sclerotigenus, Hydnum henningsii, Irpex lacteus, Polyporus sulphreus, Polyporus betreus, Polustictus hirfutus, Trametes vitata, Irpex consolus, Lentines lepideus, Poria vaporaria, Fomes pinicola, Lenzites styracina, Merulius lacrimans, Polyporus palstris, Polyporus annosus, Polyporus versicolor, Polystictus sanguineus, Poris vailantii, Puccinia graminis, Tricholome fumosum, Tricholome nudum, Trametes sanguinea, Polyporus schweinitzil FR, Conidiophora carebella and Acremonium sp.

Examples of prokaryotic microorganisms capable of producing a cellulase include:

Bacillus hydrolyticus, Cellulobacillus mucosus, Cellulobacillus myxogenes, Cellulomonas sp., Cellvibrio fulvus, Cellvibrio vulgaris, Clostridiumn thermocellulaseum, Clostridiumn thermocellum, Corynebacterium sp., Cytophaga globulosa, Pseudomonas fluoroescens var. cellulisa, Pseudomonas solanacearum, Bacterioides succinogenes, Ruminococcus albus Ruminococcus flavefaciens, Sorandium composition, Butyrivibrio, Clostridium sp., Xanthomonas cyamopsidis, Sclerotium bataticola, Bacillus sp., Thermoactinomyces sp., Actinobifida sp., Actinomycetes sp. and Streptomyces sp.

While the cellulase preparation of the present invention may consist of the above cellulase component only, it may further comprise suitable additives. Examples of the additives include pH adjustors (for example, phosphates and acetates) and stabilizers (for example, glycerol and sucrose).

The method for treating a cellulosic fiber with the cellulase preparation of the present invention is not particularly limited so far as the cellulase component is brought into contact with the cellulosic fiber. Preferably, after the cellulase preparation of the present invention is dissolved in water, the aqueous solution is brought into contact with the cellulosic fiber. Alternatively, the cellulase component may be immobilized on a suitable carrier (for example, a particulate or flaky carrier) and brought as the immobilized enzyme into contact with the cellulosic fiber.

EXAMPLES

The present invention will now be described in more detail with reference to the following examples, though it is not limited to these examples only.

In the following examples, the adsorptivity to crystalline cellulose, Avicelase activity and CMCase activity were measured as follows.

Evaluation on adsorptivity to crystalline cellulose

Eight (8) mg of a cellulase is mixed with a suspension of 0.2 g of microcrystalline cellulose (Avicel SF™; manufactured by Asahi Kasei Kogyo K.K.) in 4 ml of a buffer (0.05 M buffer: an acetate buffer for a pH range of from 3 to 6 and a phosphate buffer for a pH range of from 6 to 7). The mixed solution is placed in a L-shaped test tube (a round bottom) and shaken at a shaking rate of 60 times/min for 30 min by means of a Monod type shaker while maintaining the temperature at 25°C. Subsequently, the suspension is centrifuged at 3000 rpm for 10 min. As a result, the cellulase having an adsorptivity to crystalline cellulose is precipitated in such a state that it is adsorbed to crystalline cellulose. The cellulase not having an adsorptivity to crystalline cellulose is fractionated as a supernatant. The protein content of the adsorbed cellulase can be determined from a difference in protein content between the original cellulase and the cellulase recovered in the supernatant by using the UV absorption method, Lowry method, Bradford method and other methods.

Avicelase activity

A 2% (w/v) suspension of microcrystalline cellulose (Avicel SF™; manufactured by Asahi Kasei Kogyo K.K.) in distilled water is used as a substrate suspension. After 2.0 ml of a 0.05 M buffer adjusted to a suitable pH value is added to 2.5 ml of the substrate suspension, the mixture is placed in an L-shaped test tube (a round bottom). The test tube is maintained at a measuring temperature for 10 min.

An enzyme sample to be analyzed is dissolved in distilled water. 0.5 ml of the solution is added to the above test tube. The test tube is accurately shaken at a shaking rate of 60 times/min for 30 min by means of a Monod type shaker while maintaining the test tube at the measuring temperature, thereby allowing a

6

reaction to proceed. Thereafter, 0.5 ml of a 0.5 N sodium hydroxide solution is added thereto to stop the reaction.

Separately, 0.5 ml of a 0.5 N sodium hydroxide solution is added to a solution of the same kind as described above before the reaction to inhibit the progress of the reaction. This solution is used as a control.

After stopping the reaction, the reaction mixture is centrifuged (3000 rpm x 10 min). The reducing sugar content of the supernatant is measured by the following DNS (3,5-dinitrosalicylic acid) method. The DNS reagent is prepared according to a method specified in IUPAC.

A short test tube (18mm∅ X 130mm) is charged with 0.5 ml of the supernatant is placed as a sample and then 1.5 ml of a DNS reagent solution. The test tube is allowed to stand in boiling water for 5 min while preventing evaporation by a glass cap to heat the test tube, thereby developing a color. After the reaction mixture is sufficiently cooled with running water, 4 ml of distilled water is added thereto. Then, the absorbance (540 nm) of the mixture is measured.

The reducing sugar content of the reaction mixture can be read from a calibration curve. The calibration curve is prepared using glucose anhydride (guaranteed reagent) in concentrations of 0, 0.2, 0.4, 0.6, 0.8 and 1.0 mg/ml.

This calibration curve is used to determine the difference in reducing sugar content between the sample and the control. The difference is regarded as the formed reducing sugar content.

The Avicelase activity (U/g) can be determined from the formed reducing sugar content and the enzyme content of the enzyme sample solution used in the reaction according to the following equation:

$$\text{Avicelase activity (U/g)} = \frac{\text{formed reducing sugar content (mg)}}{\text{enzyme content (mg)}} \times 11 \times 1000 \times 1000 / 180 / 30$$

(1U/g = 1 $\mu$mol/min/g)

CMCase activity

A 2% (w/v) solution of CMC (carboxymethyl cellulose sodium manufactured by Tokyo Chemical Industry Co., Ltd.; n = 500) dissolved in a 0.1 M buffer adjusted to suitable pH is used as a substrate solution. 0.5 ml of the substrate solution is placed in a short test tube and maintained at a measuring temperature for 10 min.

After an enzyme sample to be analyzed is dissolved in distilled water, 0.5 ml of the solution is added to the test tube. The test tube is allowed to stand accurately for 30 min, thereby allowing a reaction to proceed. 3.0 ml of a DNS reagent solution is added thereto to stop the reaction.

Separately, 3.0 ml of a DNS reagent solution is added to a solution of the same kind as described above before the reaction to inhibit the progress of the reaction. This solution is used as a control.

After stopping the reaction, the reaction mixture is allowed to stand in boiling water for 5 min while preventing evaporation by a glass cap to heat the test tube, thereby developing a color in the whole sample. After the reaction mixture is sufficiently cooled with running water, 8 ml of distilled water is added. The absorbance (540 nm) of the mixture is then measured.

The reducing sugar content of the reaction mixture can be read from a calibration curve. The calibration curve is prepared using glucose anhydride (guaranteed reagent) in concentrations of 0, 0.2, 0.4, 0.6, 0.8 and 1.0 mg/ml.

This calibration curve is used to determine the difference in reducing sugar content between the sample and the control. The difference is regarded as the formed reducing sugar content.

The CMCase activity (U/g) can be determined from the formed reducing sugar content and the enzyme content of the enzyme sample solution used in the reaction according to the following equation:

$$\text{CMCase activity (U/g)} = \frac{\text{formed reducing sugar content (mg)}}{\text{enzyme content (mg)}} \times 2 \times 1000 \times 1000 / 180 / 30$$

(1U/g = 1 $\mu$mol/min/g)

Example 1 Fractionation of cellulase derived from Trichoderma viride

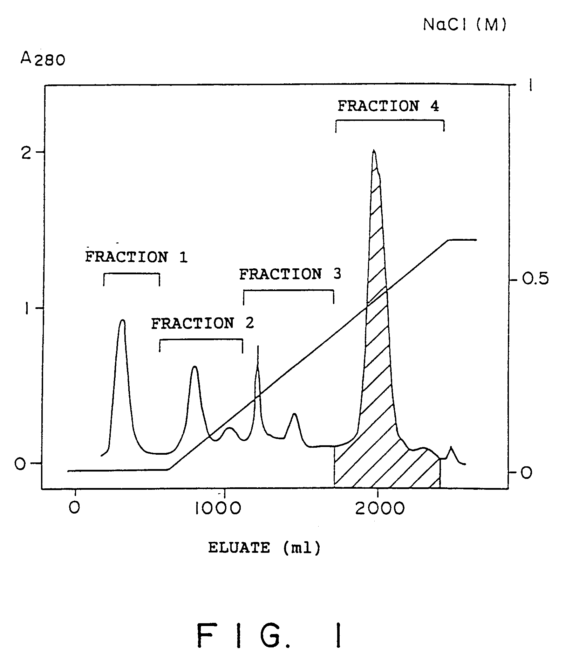

A commercially available cellulase derived from Trichoderma viride (Meiji Cellulase TP60™ manufactured by Meiji Seika Kaisha, Ltd.) was dissolved in an amount of 10% (w/v) in water. The aqueous solution was applied to an anion-exchange column (monoQ (60/100) manufactured by Pharmacia) and subjected to elution and fractionation using a 50 mM acetate buffer (pH: 5.8) with an aqueous NaCl solution of 0 to 0.6 M density gradient. The fractionation pattern was as shown in Fig. 1. The fraction collected in the density gradient range of from 0.35 to 0.6 M was designated as fraction No. 4.

Then, the Avicelase activity and the CMCase activity of a component having an adsorptivity to crystalline cellulose in the fraction No. 4 were measured according to the above method. Specifically, the Avicelase activity and the CMCase activity of a component not having an adsorptivity to crystalline cellulose were determined. The difference in Avicelase activity and CMCase activity between the whole cellulase and the component not having an adsorptivity to crystalline cellulose was determined to obtain the above value. The measurement was carried out under conditions of a temperature of 45°C and a pH value of 4.5.

As a result, the Avicelase activity/CMCase activity value of the fraction No. 4 was 0.46. On the other hand, the Avicelase activity/CMCase activity value of the unpurified cellulase was 0.058.

Further, according to SDS-PAGE and electrofocusing, the fraction No. 4 contained a cellulase having a molecular weight of about 59 kd and an isoelectric point in the range of from about 3.8 to 4.8.

Example 2 Fiber (yarn) strength test

The cellulase of the fraction No. 4 obtained in Example 1 was added to the commercially available cellulase used in Example 1 to prepare cellulases having Avicelase activity/CMCase activity values of 0.107, 0.164, 0.250 and 0.480 U/g.

Twelve (12) oz of jean was unraveled and cut accurately to an even length of 11 cm as test yarns.

After the test yarns were placed five by five in test tubes, a buffer (0.1 M acetate buffer, pH 4.5) and the cellulase prepared above in such an amount as to cause a loss in weight of fiber of 5% determined by a preliminary test were added thereto. Further, deionized water was added to the test tubes to a final volume of 5 ml. The test tubes were hermetically sealed with a glass cap and were allowed to stand in a water bath of 45°C for 16 hr.

After the completion of the reaction, the test yarns were washed in deionized water by repeating stirring for 10 sec twice with a voltex mixer. After centrifugation (3000 rpm x 10 min), the test yarns were dried at 50°C for 30 min, allowed to stand at room temperature for 30 min or more and weighed to determine the loss in weight. All the test yarns had a loss in weight of 5%.

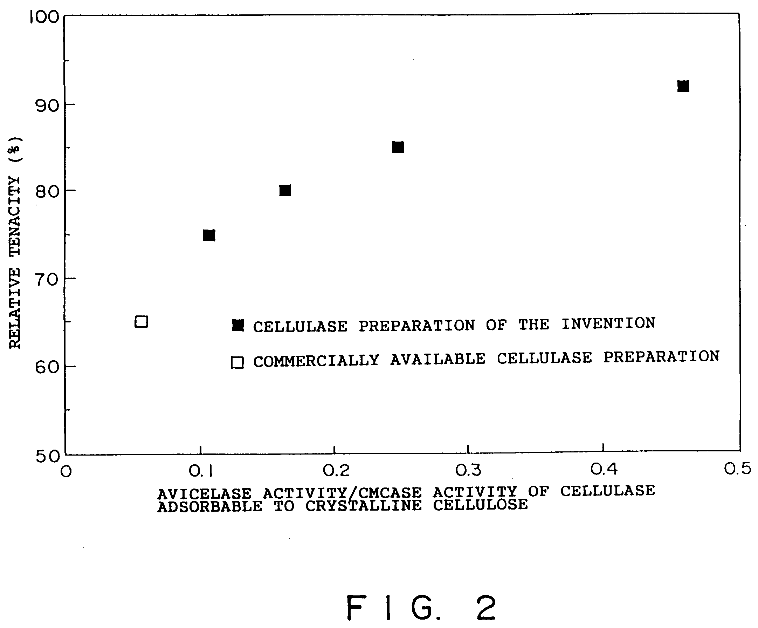

Subsequently, the tenacity of the test yarns was measured. The measurement was carried out using a rheometer (NRM-2010J-CW manufactured by Fudow Kogyo Co., Ltd. ) under conditions of a tensile speed of 5 cm/min, 2 kg range and an exclusive adapter No. 19 (a universal tensile type). The results were as shown in Fig. 2.

Example 3 Decolorization and fiber strength test

Twelve (12) oz of jean was cut to a size of 12 x 4 cm and used as a test cloth. All side of the test cloth was stitched for the purpose of preventing fray.

The test cloth was reacted with the cellulase of the fraction No. 4 obtained in Example 1 as follows. Separately, as a control, the same reaction was carried out, except that the cellulase used was the commercially available cellulase used in Example 1.

At the outset, a buffer (pH = 4.5) and the cellulase in such an amount as to cause a loss in weight of fiber of 5% determined by a preliminary test were placed in a nylon bag (designation S-1001 manufactured by Cosmo-Bio Co., Ltd. cut to a size of 10 x 20 cm). The test tube was charged with deionized water of a final volume of 50 ml and was heat-sealed. The nylon bag was put under hot water of 45°C and allowed to stand for 23 hr, thereby allowing a reaction to proceed.

After the completion of the reaction, the test cloth was washed in deionized water with a stirrer for 10 min, dehydrated in a dehydrating tub of a domestic washer for 1 min and dried at 50°C.

In all the test cloths, the loss in weight was 5%.

After the test cloth was unraveled, the yarn was taken out and subjected to measurement of the tenacity of the test cloth after drying according to a method described in Example 2. In the measurement of the tenacity, 5 yarns were used for every test group. The relative residual tenacity was calculated using the average value.

The degree of decoloring of the test cloth was measured as follows.

A color analyzer TC-1800M K2 (manufactured by Tokyo Denshoku Co., Ltd.) was used to measure the L value (brightness) of Lab color specification system for 5 points of the test cloth. The measurement was carried out under conditions of colorimetry by measurement of reflected light, visual field of 2° and reference light D65/2, C/2 and A/2. The average value of three points with the maximum value and the minimum value being excluded was determined. The degree of decoloring was determined as a ΔL value which is an increase in L value relative to the control test cloth (an increase in whiteness).

As a result, in a degree of decoloring of 1.6 in terms of ΔL value, the relative residual tenacity was 75% for the treatment with the cellulase of the fraction No. 4, whereas in the control, the relative residual tenacity was as low as 53%.

Example 4 Fiber (cloth) strength test

A cotton broad No. 40 was used as a test cloth. The cellulase of the fraction No. 4 prepared in Example 1 was used as a cellulase.

The test cloth was reacted with the cellulase as follows. Specifically, 5 sheets of the test cloth and a buffer (pH = 4.5) were placed in the same nylon bag as used in Example 3. The cellulase in such an amount as to cause a loss in weight of fiber of 5% determined by a preliminary test was added to the bag. The nylon bag was allowed to stand at 45°C for 16 hr, thereby allowing a reaction to proceed.

After the completion of the reaction, the test cloth was washed with running water for 5 min, dehydrated in a dehydrating tub of a domestic washer for 5 min and dried at 50°C.

As a control, the same reaction was carried out, except that the cellulase used was the commercially available cellulase used in Example 1.

In all the test cloths, the loss in weight was 5%.

The tenacity and the tearing strength of the cloth were measured according to JIS (testing method for general fabrics (JIS L 1096, measuring methods for tensile strength and elongation (method A, strip method, width 5 cm) and measuring method for tearing strength (method D, pendulum method)).

The results were as shown in the following tables.

Table 1

| Tenacity (kgf) | | |
|---|---|---|
| | Longitudinal (relative value) | Transverse (relative value) |
| Example | 37.0 (80.4%) | 27.2 (75.1%) |
| Control | 32.7 (71.1%) | 18.1 (50.0%) |
| no enzyme | 46.0 (100%) | 36.2 (100%) |

Table 2

| Tear strength (kgf) | | |
|---|---|---|
| | Longitudinal (relative value) | Transverse (relative value) |
| Example | 0.73 (56.6%) | 0.70 (81.4%) |
| Control | 0.66 (51.2%) | 0.63 (73.3%) |
| No enzyme | 1.29 (100%) | 0.86 (100%) |

Example 5 Cellulase derived from Acremonium

Acellulase derived from a strain (FERM P-6867) belonging to Acremonium was fractionated in the same manner as in Example 1 to collect a fraction having a large Avicelase activity/CMCase activity value. The Avicelase activity/CMCase activity value of the original cellulase was 0.076, whereas that of the fraction was 0.33.

The procedure of Example 2 was repeated, except that the fraction thus obtained was used. As a result, the relative residual tenacity was 85% in a loss in weight of 2.5%. Incidentally, when the original cellulase was used, the relative residual tenacity was 75%.

**Claims**

1. A cellulase preparation characterized in that when a fiber is treated with said cellulase preparation, the treated fiber has a relative residual tenacity of not less than 75% per yarn count in a loss in weight of 5%.

2. The cellulase preparation according to claim 1, which is used in a loss in weight treatment of a fiber.

3. The cellulase preparation according to claim 1, which comprises a cellulase having an adsorptivity to crystalline cellulose and an Avicelase activity to CMCase activity ratio (Avicelase activity/CMCase activity value) of not less than 0.1.

4. The cellulase preparation according to claim 1, wherein said cellulase has an adsorptivity to crystalline cellulose in an acidic or neutral region.

5. The cellulase preparation according to claim 1, wherein said cellulase has an activity in a pH range of from 3 to 8.

6. The cellulase preparation according to claim 1, wherein said cellulase is derived from a microorganism.

7. The cellulase preparation according to claim 6, wherein said cellulase is derived from an eucaryotic microorganism.

8. The cellulase preparation according to claim 7, wherein said eucaryotic microorganism belongs to the genus Trichoderma.

9. The cellulase preparation according to claim 8, wherein said cellulase has the following properties:
   (a) average molecular weight: about 59 kd (by SDS-PAGE),
   (b) isoelectric point: about 3.8 to 4.2,
   (c) Avicelase activity: not less than 100 units/g of the total solid matter, and
   (d) optimal pH: about 4.2 to 4.8.

10. A method for treating a fiber, which comprises bringing a cellulase preparation according to any one of claims 1 to 9 into contact with a cellulosic fiber.

11. The method for treating a fiber according to claim 10, wherein said cellulase preparation is brought into contact with said fiber at a temperature in the range of from 30 to 80°C.

F I G. 1

FIG. 2

EP 0 636 740 A1

INTERNATIONAL SEARCH REPORT

International application No.

PCT/JP94/00199

| A. | CLASSIFICATION OF SUBJECT MATTER |
|---|---|

Int. Cl$^5$   D06M16/00, C12N9/42

According to International Patent Classification (IPC) or to both national classification and IPC

| B. | FIELDS SEARCHED |
|---|---|

Minimum documentation searched (classification system followed by classification symbols)

Int. Cl$^5$   D06M16/00, C12N9/42

Documentation searched other than minimum documentation to the extent that such documents are included in the fields searched

Electronic data base consulted during the international search (name of data base and, where practicable, search terms used)

BIOSIS WPI, WPI/L

| C. | DOCUMENTS CONSIDERED TO BE RELEVANT | |
|---|---|---|
| Category* | Citation of document, with indication, where appropriate, of the relevant passages | Relevant to claim No. |
| A | JP, A, 5-214664 (Asahi Chemical Industry Co., Ltd.), August 24, 1993 (24. 08. 93), (Family: none) | 1, 2 |
| A | JP, A, 58-36217 (Asahi Chemical Industry Co., Ltd.), March 3, 1983 (03. 03. 83), (Family: none) | 1, 2 |
| A | Agric. Biol. Chem., Vol. 52, No. 10 (1988), T. Yamanobe et. al. "Purification and Some Properties of a Microcrystalline Cellulose-hydrolyzing Ewzyme (Avicelase II) from Fungal Strain Y-94" | 3-6 |

☐ Further documents are listed in the continuation of Box C.        ☐ See patent family annex.

| * | Special categories of cited documents: |
|---|---|
| "A" | document defining the general state of the art which is not considered to be of particular relevance |
| "E" | earlier document but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| April 21, 1994 (21. 04. 94) | May 17, 1994 (17. 05. 94) |

| Name and mailing address of the ISA/ | Authorized officer |
|---|---|
| Japanese Patent Office | |
| Facsimile No. | Telephone No. |

Form PCT/ISA/210 (second sheet) (July 1992)